# FASCICULE DE BREVET EUROPEEN

(11) **EP 2 273 943 B1**
(45) Date de publication et mention de la délivrance du brevet: **18.03.2015**
(21) Numéro de dépôt: 08787864.1
(22) Date de dépôt: 27.03.2008
(51) Int. Cl.: A61B 17/80, A61B 17/86, A61B 17/88

(54) **SYSTEME D'ARTHRODESE DE CHEVILLE D'UN PATIENT HUMAIN**
ARTHRODESESYSTEM FÜR DAS FUSSGELENK EINES MENSCHLICHEN PATIENTEN
ARTHRODESIS SYSTEM FOR THE ANKLE OF A HUMAN PATIENT

(43) Date de publication de la demande: 19.01.2011
(73) Titulaire: Newdeal, 69800 Saint-Priest (FR)
(72) Inventeur: HINTERMANN, Beat, CH-4410 Liestal (CH)
(74) Mandataire: Weber, Jean-François
(86) Numéro de dépôt international: PCT/FR2008/000424
(87) Numéro de publication internationale: WO 2009/118461

(56) Documents cités:
- WO-A-2007/103333
- WO-A-2007/127994
- WO-A-2007/131287
- US-A- 5 853 413
- US-A1- 2004 116 930
- US-A1- 2006 004 380
- US-A1- 2006 235 427
- US-A1- 2007 100 338

## Description

### DOMAINE TECHNIQUE

La présente invention se rapporte au domaine technique général des dispositifs chirurgicaux destinés à assurer la solidarisation de deux parties osseuses d'une articulation, en vue par exemple de réaliser une arthrodèse.

La présente invention concerne plus précisément un système pour immobiliser l'articulation entre le tibia et le talus d'un patient humain.

### TECHNIQUE ANTERIEURE

Dans le cas de certaines pathologies survenant au niveau de l'articulation entre le tibia et le talus (ostéoarthrite primaire, ostéoarthrite post-traumatique, arthrite de la cheville) ou à la suite de l'échec d'une arthroplastie totale de la cheville, il peut s'avérer nécessaire de pratiquer une arthrodèse afin de fusionner les deux os (talus et tibia) entre eux. De manière générale, les arthrodèses sont des opérations délicates, puisqu'elles bloquent une articulation dans une position définie de manière irréversible. C'est ainsi que l'arthrodèse de l'articulation entre le tibia cette talus revêt un caractèr hautement important, dans la mesure où cette articulation intervient de manière essentielle dans le cycle de la marche d'un être humain. On comprend alors qu'il s'avère essentiel, compte-tenu en particulier des cycles de charge auxquels est soumise l'articulation entre le tibia et le talus, que l'arthrodèse de cette articulation soit réalisée de la manière la moins invasive et la plus précise possible, pour éviter toute gêne ultérieure.

Jusqu'à présent, les arthrodèses de la cheville sont réalisées le plus souvent à l'aide de plaques de fixation osseuse pourvues de lumières destinées à recevoir des vis de fixation pour assurer la solidarisation de la plaque avec les os à fusionner.

On connaît ainsi des plaques destinées à être fixées sur le côté latéral du tibia et du talus. Ces plaques sont toutefois généralement difficiles à poser, en particulier à cause de la gêne opératoire occasionnée par la présence du péroné. Ces plaques connues présentent de surcroît une forme qui ne leur permet pas d'épouser au plus près les contours osseux, ce qui engendre un risque de gêne et de complications ultérieures pour le patient, et ne facilite pas l'intervention chirurgicale. Afin de remédier à ce problème, le chirurgien peut être conduit à effectuer des opérations de mise en forme de la plaque au moment de l'opération, par pliage manuel (à l'aide de pinces) de la plaque. De telles opérations de pliage constituent une contrainte supplémentaire importante dans le cadre de l'intervention chirurgicale, et peuvent en outre induire une fragilisation du métal au niveau de la zone de pliage, ce qui présente un inconvénient important en terme de résistance mécanique. De plus, la nécessité de prévoir des ouvertures pour le passage des vis de fixation dans la plaque complique l'opération de pliage de la plaque en raison précisément de la présence de ces ouvertures.

En définitive, les plaques connues ne permettent pas de réaliser des arthrodèses tibia/talus dans des conditions opératoires optimales et avec un risque minimal de gêne et de complications ultérieures pour le patient.

On connaît par ailleurs du document US-2007/0100338 un système conforme au préambule de la revendication 1.

### EXPOSE DE L'INVENTION

L'objet de l'invention vise en conséquence à porter remède aux différents inconvénients énumérés précédemment et à proposer un nouveau système pour immobiliser l'articulation entre le tibia et le talus d'un patient humain, qui permette de réaliser une arthrodèse dans des conditions opératoires optimales tout en permettant d'obtenir une fusion osseuse particulièrement stable, résistante, précise et confortable pour le patient.

Un autre objet de l'invention vise à proposer un nouveau système pour immobiliser l'articulation entre le tibia et le talus d'un patient humain qui soit particulièrement confortable pour le patient, en toutes circonstances.

Un autre objet de l'invention vise à proposer un nouveau système pour immobiliser l'articulation entre le tibia et le talus d'un patient humain qui soit particulièrement facile et rapide à mettre en place, et qui procure une fusion osseuse particulièrement fiable.

Un autre objet de l'invention vise à proposer un nouveau système pour immobiliser l'articulation entre le tibia et le talus d'un patient humain qui favorise le processus d'ostéosynthèse entre le tibia et le talus.

Un autre objet de l'invention vise à proposer un nouveau système pour immobiliser l'articulation entre le tibia et le talus d'un patient humain qui permette de favoriser l'ostéosynthèse de manière particulièrement simple, rapide et fiable.

Les objets assignés à l'invention sont atteints à l'aide d'un système conforme à l'objet de la revendication 1.

### DESCRIPTIF SOMMAIRE DES DESSINS

D'autres avantages et objets de l'invention apparaîtront plus en détails à la lecture de la description qui suit, et à l'aide des dessins annexés fournis à titre purement explicatif et non limitatif, dans desquels :
- La figure 1 illustre, selon une vue de face (vue ventrale), les plaques antéro-letérale et antéro-médiale du système conforme à l'invention positionnées sur le tibia et le talus droits d'un patient humain pour immobiliser l'articulation entre le tibia et le talus.
- La figure 2 est identique à la figure 1, à la différence qu'il ne s'agit pas d'une vue de face (vue ventrale) mais d'une vue de côté latérale.
- La figure 3 est identique aux figures 1 et 2 à la différence près qu'il s'agit d'une vue de côté médiale.

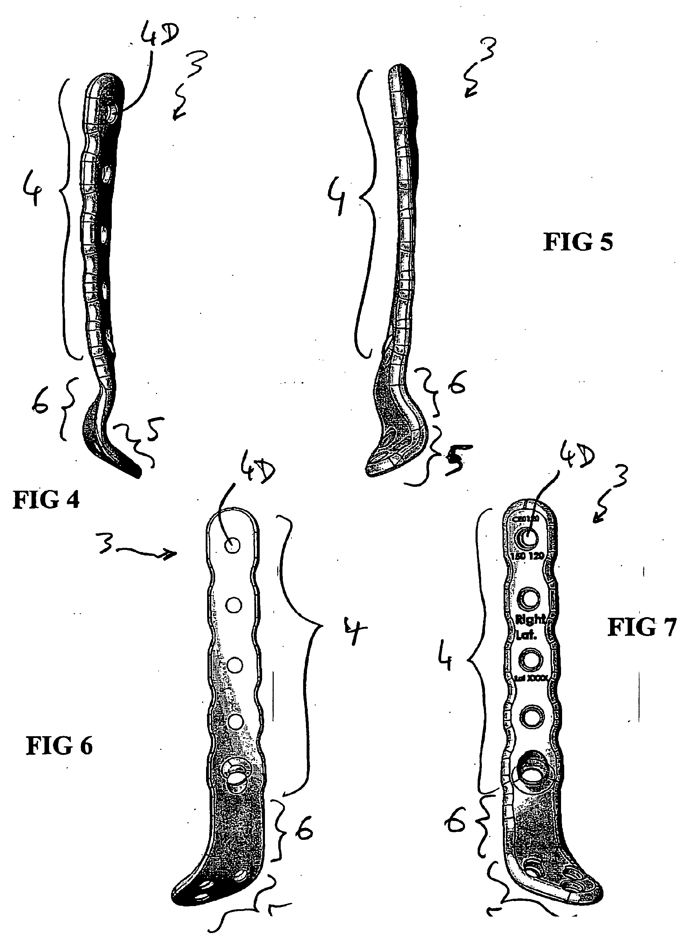

- La figure 4 illustre, selon une vue de côté latérale, la plaque antéro-latérale des figures 1 à 3.
- La figure 5 illustre, selon une vue de côté médiale, la plaque antéro-latérale de la figure 4.
- La figure 6 illustre, selon une vue arrière (vue dorsale), la plaque antéro-latérale des figures 4 et 5.
- La figure 7 illustre, selon une vue de face (vue ventrale), la plaque antéro-latérale des figures 4 à 6.
- La figure 8 illustre, selon une vue de face (vue ventrale), la plaque antéro-médiale des figures 1 à 3.
- La figure 9 illustre, selon une vue de profil arrière, la plaque antéro-médiale de la figure 8.
- La figure 10 illustre, selon une vue latérale, la plaque antéro-médiate des figures 8 et 9.
- La figure 11 illustre, selon une vue médiale, la plaque antéro-médiale des figures 8 à 10.
- La figure 12 illustre, selon une vue arrière (vue dorsale), la plaque antéro-médiale des figures 8 à 11.
- La figure 13 illustre un couple de plaques antéro-latérale et antéro-médiale faisant partie d'un système conforme à l'invention pour réaliser l'immobilisation de l'articulation entre le tibia et le talus droit d'un patient humain.
- La figure 14 illustre, selon une vue de face, un couple de plaques antéro-latérale et antéro-médiale faisant partie d'un système conforme à l'invention destinées à immobiliser l'articulation entre le tibia et le talus gauche d'un patient humain.

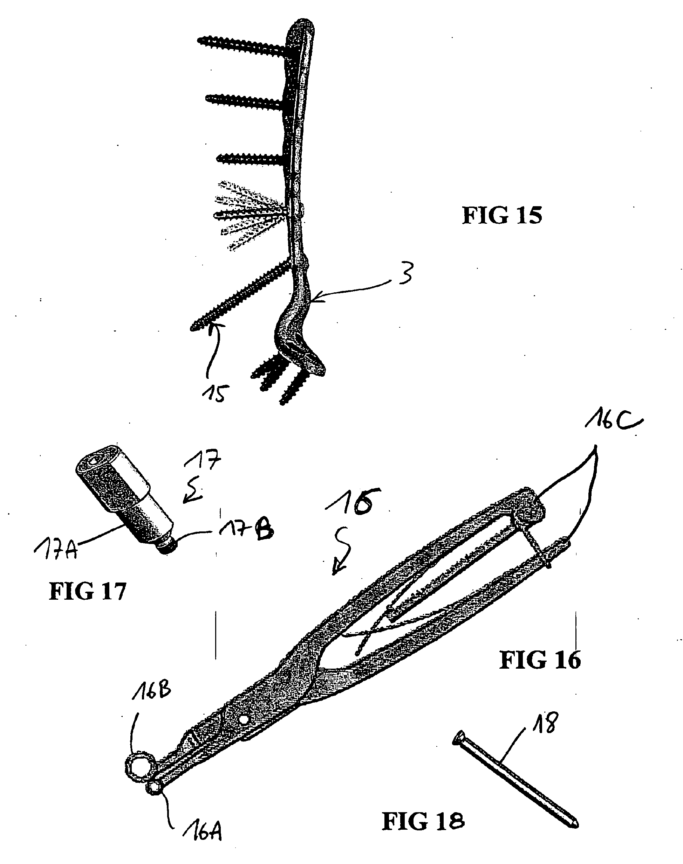

- La figure 15 illustre, selon une vue de côté, la plaque antéro-latérale de la figure 14 coopérant avec des vis d'ancrage osseux.
- La figure 16 illustre, selon une vue de dessus, un pince formant moyen de compression de l'articulation entre le tibia et le talus.
- La figure 17 illustre, selon une vue en perspective, un canon de fixation et de guidage destiné à être associé à un premier mors de la pince de la figure 16.
- La figure 18 illustre, selon une vue en perspective, une vis de fixation destiné à être associée au deuxième mors de mors de la pince de la figure 16.
- La figure 19 illustre la coopération de la pince de la figure 16 avec d'une part le canon de la figure 17 et d'autre part la vis de la figure 18.
- La figure 20 illustre la mise en oeuvre du moyen de compression de la figure 19 en coopération avec la plaque antéro-médiale de la figure 14.
- Les figures 21 et 22 illustrent deux exemples de vis (à têtes respectivement tronconique et sphérique) et de contre-vis correspondantes (systèmes de type Surfix ®) utilisables assurer la fixation osseuse des plaques antéro-latérale et antéro-médiale.

### MEILLEURE MANIERE DE REALISER L'INVENTION

L'invention concerne un système pour immobiliser l'articulation entre le tibia 1 et le talus 2 d'un patient humain, en vue de réaliser une arthrodèse de la cheville.

Le système conforme à l'invention constitue donc avantageusement un système d'arthrodèse de cheville.

Le système conforme à l'invention comprend au moins une plaque antéro-latérale 3 destinée à être fixée à la fois sur la face antérieure (ventrale) du tibia 1 et sur la face antérieure du talus 2.

La plaque antéro-latérale 3 est une plaque destinée à être implantée chirurgicalement.

Elle présente un caractère rigide et est de préférence réalisée en un matériau dur et rigide comme l'acier inoxydable, le titane ou tout autre matériau métallique de grade chirurgical.

La plaque antéro-latérale 3 comprend elle-même une patte tibio-latérale 4 et une languette talo-latérale 5 reliées par une zone de liaison 6. En d'autres termes, la patte tibio-latérale 4 se prolonge par la zone de liaison 6, laquelle se prolonge elle-même par la languette talo-latérale 5. La plaque antéro-latérale 3 présente de préférence une forme globale sensiblement allongée, comme illustré aux figures, c'est-à-dire que la longueur globale de la plaque antéro-latérale 3 est nettement supérieure à la fois à sa largeur et à son épaisseur.

Conformément à l'invention, lesdites patte tibio-latérale 4, languette talo-latérale 5 et zone de liaison 6 sont conformées pour à la fois :
- qu'au moins une portion de la patte tibio-latérale 4 repose sensiblement contre la face latérale antérieure 1A du tibia 1 située vers l'extérieur du corps du patient par rapport à la crête tibiale antérieure 1 B,
- et que la languette talo-latérale 5 repose sensiblement contre le talus 2, et de préférence contre le col 2A du talus 2.

En d'autres termes, la patte tibio-latérale 4, la languette talo-latérale 5 et la zone de liaison 6 sont positionnées, orientées et dimensionnées les unes par rapport aux autres pour que lorsque la plaque antéro-latérale 3 est installée dans sa position d'implantation définitive, au sein du corps du patient, la patte tibio-latérale 4 épouse sensiblement la face latérale antérieure 1A du tibia 1, tandis que la languette talo-latérale 5 est sensiblement plaquée contre le talus 2, et de préférence contre le col 2A du talus 2.

La plaque antéro-latérale 3 est ainsi conçue pour être positionnée en regard de l'extrémité distale du tibia 1, et être plaquée contre la face antérieure latérale 1A de ce dernier.

Avantageusement, et comme illustré aux figures, la plaque antéro-latérale 3 est conformée pour que lorsqu'elle se trouve dans sa position finale d'implantation, sa patte tibio-latérale 4 s'étende sensiblement contre la crête tibiale antérieure 1B, parallèlement à cette dernière. La patte tibio-latérale 4 se trouve ainsi plaquée contre la portion de la face latérale antérieure 1A qui se trouve immédiatement à proximité de la crête tibiale antérieure 1B, la portion en question de la face latérale antérieure 1A présentant un caractère sensiblement plat qui permet d'assurer une fixation stable et efficace de la patte tibio-latérale 4 au tibia 1, grâce à des moyens qui seront décrits plus en détails dans ce qui suit.

De préférence, la zone de liaison 6 de la plaque antéro-latérale 3 est destinée à venir en regard d'une portion au moins du pilon tibial 1C, lequel forme un renflement osseux au niveau de l'extrémité distale du tibia 1. Afin de favoriser la tolérance de la plaque antéro-latérale 3 par le patient, et donc le confort de ce dernier, la zone de liaison 6 de la plaque antéro-latérale 3 présente de préférence une forme sensiblement complémentaire de celle de la portion du pilon tibial 1C qu'elle recouvre.

De préférence, la zone de liaison 6 de la plaque antéro-latérale 3 présente une forme sensiblement incurvée de manière à suivre le contour du pilon tibial 1C, comme illustré aux figures. Ainsi, la plaque antéro-latérale 3 conforme à l'invention présente une forme générale anatomique, c'est-à-dire qu'elle est conformée pour suivre au plus près l'anatomie des structures osseuses auxquelles elle est destinée à être attachée. Grâce à cette caractéristique, la plaque antéro-latérale 3 est plus facilement supportée par le patient et est plus facile à poser pour le chirurgien.

La mise en place chirurgicale de la plaque antéro-latérale 3 est en outre grandement facilitée par le positionnement spécifique retenu, c'est-à-dire le positionnement sur la face latérale antérieure 1A du tibia 1. En effet, ce positionnement facilite le travail du chirurgien car il ne nécessite qu'une voie d'abord antérieur.

Une telle voie d'abord antérieur procure une meilleure visibilité que les voies latérale, postérieure ou médiale, il ne nécessite qu'une incision courte par rapport aux autres voies précitées et la pose de la plaque est facilitée car la voie antérieure n'est pas obstruée par des obstacles anatomiques comme le péroné (en voie latérale), le tendon d'Achille (en voie postérieure) ou la malléole (en voie médiale). En outre, il est déjà connu d'utiliser la voie chirurgicale antérieure pour poser des prothèses totales de cheville. Il est donc tout à fait intéressant de proposer à ces mêmes chirurgiens une voie d'abord antérieur pour la réalisation d'une arthrodèse talo-tibiale, et ce d'autant plus que ladite arthrodèse peut intervenir à la suite d'un échec de pose de prothèse totale de cheville. En définitive, l'invention a identifié un positionnement anatomique très précis pour la plaque antéro-latérale 3, et propose une géométrie très spécifique de la plaque antéro-latérale 3, qui permet de suivre au plus près l'anatomie du site particulier d'implantation et de procurer ainsi une fixation fiable, efficace et confortable.

Le système conforme à l'invention comprend en outre une plaque antéro-médiale 7. La plaque antéro-médiale 7 est une plaque destinée elle aussi à être implantée chirurgicalement, de préférence par la même voie antérieure que la plaque antéro-latérale 3. La plaque antéro-médiale 7 présente elle aussi un caractère rigide et est elle aussi de préférence réalisée en un matériau dur et rigide comme l'acier inoxydable, le titane ou tout autre matériau métallique de grade chirurgical.

La plaque antéro-médiale 7 comprend elle-même une patte tibio-médiale 8 et une languette talo-médiale 9 reliées par une zone de liaison 10. En d'autres termes, la patte tibio-médiale 8 se prolonge par la zone de liaison 10, laquelle est elle-même prolongée par la languette talo-médiale 9. De préférence, la plaque antéro-médiale 7 présente sensiblement une forme générale allongée, c'est-à-dire que sa longueur globale est nettement supérieure à la fois à sa largeur et à son épaisseur.

Lesdites pattes tibio-médiale 8, languette talo-médiale 9 et zone de liaison 10 de la plaque antéro-médiale 7 sont conformées pour à la fois :
- qu'au moins une portion de la patte tibio-médiale 8 repose sensiblement contre la face médiale antérieure 1D du tibia 1 située vers l'intérieur du corps du patient par rapport à la crête tibiale antérieure 1 B
- et que la languette talo-médiale 9 repose sensiblement contre le talus 2, et de préférence contre le col 2A du talus 2.

Ainsi, l'une des caractéristiques techniques particulièrement avantageuse du système conforme à l'invention réside dans la mise en oeuvre de deux plaques 3, 7 disposées vers la partie distale du tibia 1, au moins partiellement de part et d'autre de la crête tibiale antérieure 1B, lesdites plaques antéro-latérale 3 et antéro-médiale 7 étant destinées à être assujetties à la fois à la partie distale du tibia 1 et au talus 2. Un tel agencement s'avère procurer une fixation particulièrement stable et efficace, tout en étant particulièrement bien supporté par le patient puisque la plaque antéro-médiale 7, tout comme la plaque antéro-latérale 3, suit au plus près le contour de la structure osseuse contre laquelle elle est rapportée et à laquelle elle est destinée à être fixée.

Le système conforme à l'invention s'avère également particulièrement facile à poser, puisque la plaque antéro-médiale 7, tout comme la plaque antéro-latérale 3, peut être posée par voie d'abord antérieur, avec les avantages décrit précédemment qui en découlent. Les deux plaques antéro-latérale 3 et antéro-médiale 7 peuvent d'ailleurs être posées par le biais de la même incision antérieure.

La zone de liaison 10 de la plaque antéro-médiale 7 est destinée à venir en regard d'une portion au moins du pilon tibial 1C, ladite zone de liaison 10 de la plaque antéro-médiale 7 présentant à cet effet une forme sensiblement complémentaire de celle de ladite portion du pilon tibial 1C.

La plaque antéro-médiale 7 épouse ainsi avantageusement le contour des structures anatomiques qu'elle recouvre et auxquelles elle est destinée à être solidarisée, ce qui contribue non seulement à la fiabilité et à la stabilité de la fixation mais également au confort du patient.

Avantageusement, et comme illustré aux figures, la plaque antéro-médiale 7 est ainsi conformée pour que lorsqu'elle se trouve dans sa position finale d'implantation, sa patte tibio-médiale 8 s'étende au moins en partie sensiblement contre la crête tibiale antérieure 1B, parallèlement à cette dernière, du côté médial. La patte tibio-médiale 8 se trouve ainsi au moins en partie plaquée contre la portion de la face médiale antérieure 1D qui se trouve immédiatement à proximité de la crête tibiale antérieure 1B, la portion en question de la face médiale antérieure 1D présentant un caractère sensiblement plat qui permet d'assurer une fixation stable et efficace de la patte tibio-médiale 8 au tibia 1, grâce à des moyens qui seront décrits plus en détails dans ce qui suit.

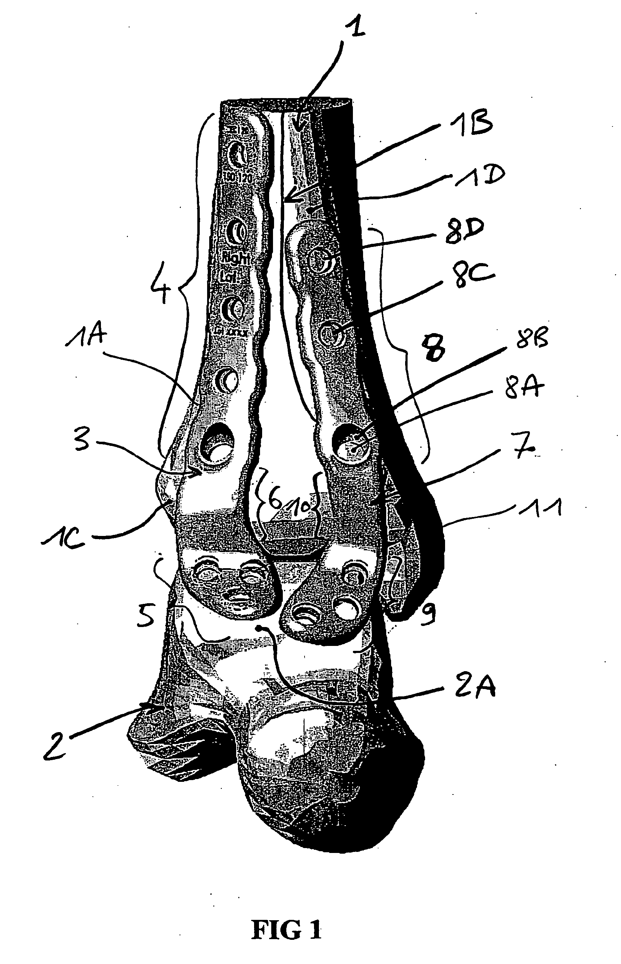

Plus précisément, et comme illustrée aux figures, la plaque antéro-médiale 7 est conçue pour être positionnée en partie à cheval sur la crête tibiale antérieure 1 B. En effet, tel que cela est bien connu et comme illustrée aux figures, la crête tibiale antérieure 1 B n'est pas rectiligne sur toute la longueur du tibia 1 et est inclinée vers la malléole interne 11 à proximité de l'extrémité distale du tibia 1. La plaque antéro-médiale 7 est ainsi avantageusement conçue pour que seule une portion terminale de sa patte tibio-médiale 8, située vers l'extrémité libre de cette dernière, soit disposée médialement par rapport à la crête tibiale antérieure 1 B. En d'autres termes, la plaque antéro-médiale 7 est de préférence conçue pour que sa languette talo-médiale 9 épouse sensiblement le col 2A du talus 2, et se prolonge par la zone de liaison 10 qui épouse l'essentiel du renflement formé par le pilon tibial 1C. La zone de liaison 10 se prolonge ensuite par une première portion (ou portion distale) de la patte tibio-médiale 8, ladite première portion étant située sous la crête tibiale antérieure 1B, ladite portion distale se prolongeant elle-même par une deuxième portion (ou portion terminale), qui de préférence s'étend sensiblement parallèlement et contre la crête tibiale antérieure 1 B (voir figure 1).

En définitive, l'invention a identifié un positionnement anatomique très précis pour la plaque antéro-médiale 7, et propose une géométrie très spécifique de la plaque antéro-médiale 7, qui permet de suivre au plus près l'anatomie du site particulier d'implantation et de procurer ainsi une fixation fiable, efficace et confortable.

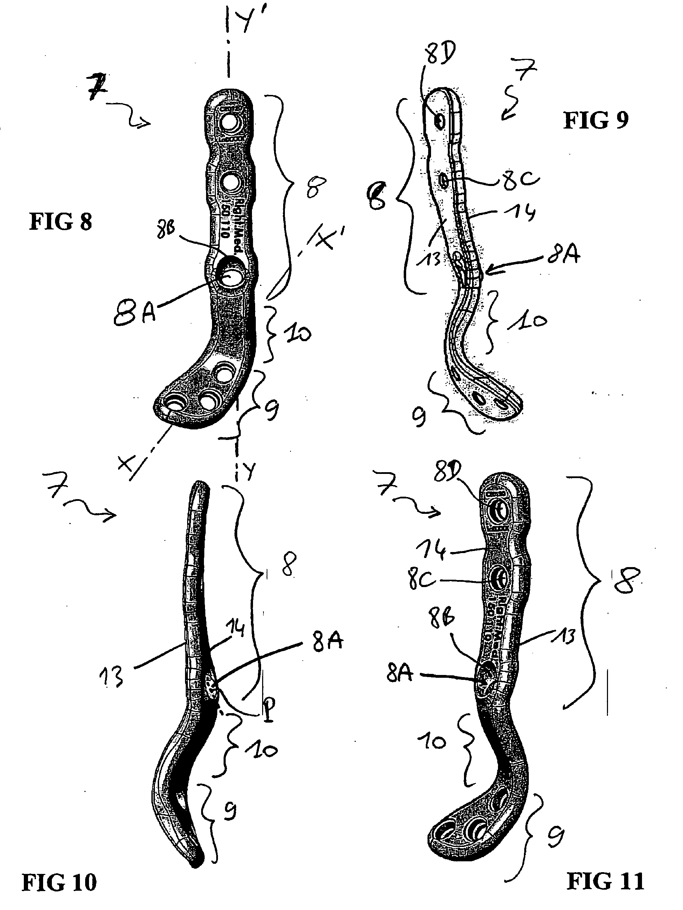

Avantageusement, tel que cela est en particulier perceptible sur les figures 9 et 10, la plaque antéro-médiale 7 présente une forme sensiblement vrillée pour suivre à la fois le contour de la face médiale antérieure 1 D du tibia 1 et du talus 2 et plus précisément du col 2A du talus 2.

De préférence, la plaque antéro-latérale 3 présente également une telle forme sensiblement vrillée, poursuivre à la fois le contour de la face latérale antérieure 1A et le contour du col 2A du talus 2.

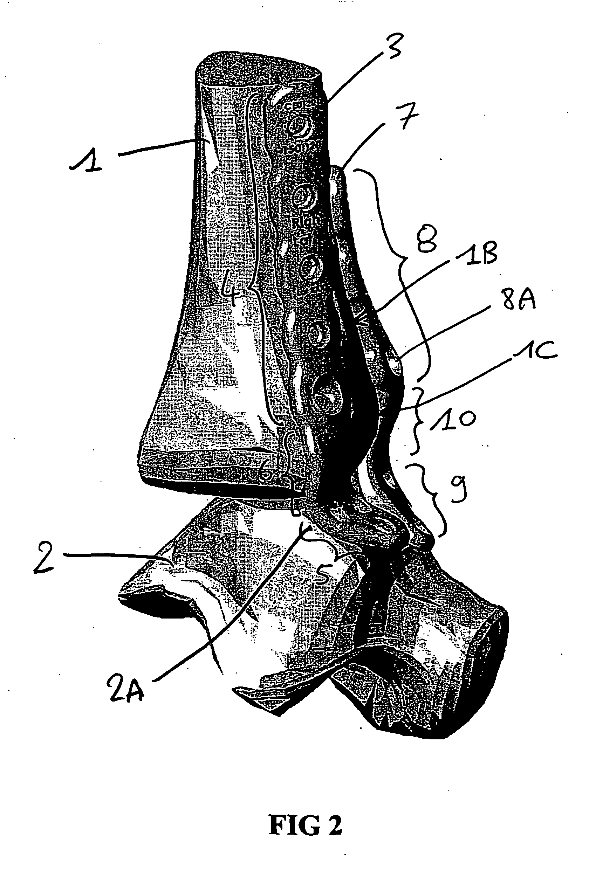

Avantageusement, la patte tibio-médiale 8 de la plaque antéro-médiale 7 présente une forme sensiblement arquée, tel que cela est en particulier visible sur les figures 2, 3 et 10, pour suivre le contour à la fois de la face médiale antérieure 1D du tibia 1 et le contour d'au moins une portion du pilon tibial 1C. En d'autres termes, la patte tibio-médiale 8 est de préférence conformée et dimensionnée pour s'étendre non seulement sur la face médiale antérieure 1D mais également pour épouser le début du renflement formé par le pilon 1C, le reste de ce renflement étant épousé par la zone de liaison 10, laquelle se prolonge par la languette talo-médiale 9.

Grâce au positionnement très particulier des plaques antéro-latérale 3 et antéro-médiale 7 identifiées dans le cadre de l'invention, il est possible d'obtenir, avec seulement deux plaques de petites dimensions, faciles à poser, une fixation extrêmement robuste stable et confortable, qui est en outre aisée à réaliser pour le chirurgien, notamment en regard des techniques connues dans l'art antérieur.

Avantageusement, et comme cela est plus particulièrement perceptible sur les figures 10 et 11, la plaque antéro-médiale 7 (tout comme de préférence la plaque antéro-latérale 3) présente, vue de profil, sensiblement une forme de cuillère, la patte tibio-médiale 8 correspondant au manche de la cuillère tandis que la zone de liaison 10 et la languette talo-médiale 9 forment la , partie concave (récipient) de la cuillère. Avantageusement, la languette talo-médiale 9 s'étend selon une direction longitudinale principale X-X' et qui est inclinée latéralement, c'est à dire vers l'extérieur du corps du patient, par rapport à la direction longitudinale principale Y-Y' d'extension de la patte tibio-médiale 8.

En d'autres termes, en vue de face (figure 8), la plaque antéro-médiale 5 présente un corps principal sensiblement rectiligne, correspondant sensiblement à la patte tibio-médiale 8 et à la zone de liaison 10, ce corps principal étant pourvu d'un élément spatulé faisant un angle non nul avec le corps principal, l'élément spatulé en question correspondant à la languette talo-médiale 9.

De préférence, la plaque antéro-latérale 3 présente une forme globalement symétrique, par rapport à la crête tibiale antérieure 1B, à la forme de la plaque antéro-médiale 7, c'est-à-dire que la languette talo-latérale 5 est avantageusement inclinée vers l'intérieur du corps du patient par rapport à la patte tibio-latérale 4.

Grâce à cette caractéristique, les languettes 5, 9 sont recentrées vers le milieu du col 2A du talus 2, ce qui permet de bénéficier d'une surface osseuse qui est non seulement efficace pour la fixation (puisque présentant en cet endroit un caractère globalement plat) mais est en outre facile à atteindre et à identifier par le chirurgien.

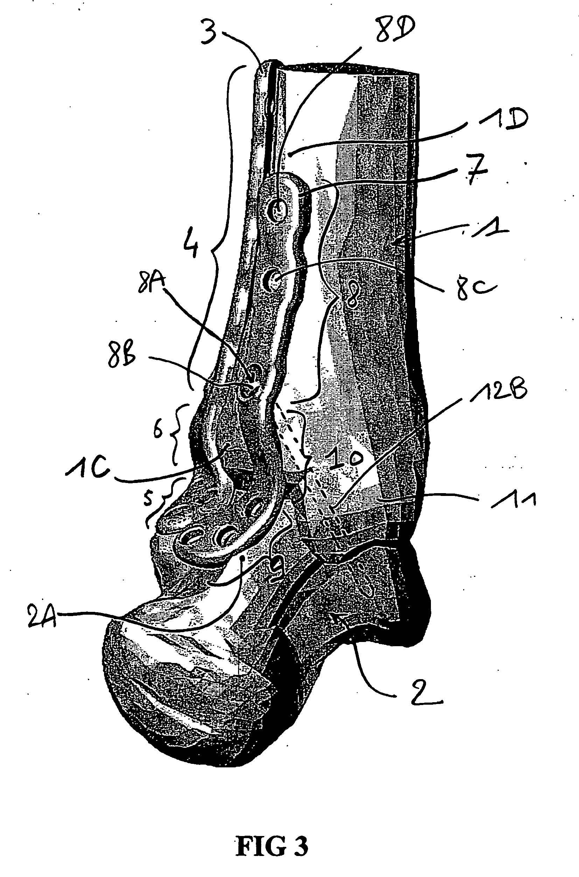

Avantageusement, le système conforme à l'invention comprend en outre au moins un premier organe d'ancrage 12 pourvu d'une tête 12A et d'une tige 12B d'ancrage osseux s'étendant à partir de ladite tête 12A, la patte tibio-médiale 8 comprenant au moins un premier orifice 8A pourvu d'une première portée 8B contre laquelle ladite tête 12A est destinée à venir en butée, ladite première portée 8B étant avantageusement conformée pour que la tige d'ancrage 12B puisse s'étendre selon une direction lui permettant de traverser à la fois le tibia 1 et le talus 2 (comme illustré en pointillés sur la figure 3), la tige 12B formant ainsi une tige d'ancrage trans-articulaire. Selon un autre mode de réalisation particulièrement avantageux, la première portée 8B du premier orifice 6A est avantageusement conformée pour que la tige d'ancrage 12B puisse s'étendre à la fois à travers le tibia 1, le talus 2 et le calcanéum (non représenté). Ainsi, en combinant une orientation adaptée de la première portée 8B du premier orifice 8A et une longueur suffisante de la tige 12B du premier organe d'ancrage 12 correspondant, il est possible de réaliser une fixation trans-articulaire solidarisant la plaque antéro-médiale 7, le tibia 1, le talus 2 et éventuellement le calcanéum.

De préférence, le premier organe d'ancrage 12 est constitué par une vis osseuse destinée à être vissée à la fois dans le tibia 1 et le talus 2, et dont la longueur est appropriée pour cela.

Avantageusement, comme illustré aux figures, la patte tibio-médiale 8 comporte une pluralité d'orifices 8A, 8C, 8D, chacun destiné à être associé à un organe d'ancrage correspondant. Avantageusement, le premier orifice 8A est positionné au plus proche de la zone de liaison 10 de la patte tibio-médiale 5, c'est à dire qu'il constitue l'orifice le plus distal de la patte tibio-médiale 8. En d'autres termes, l'orifice le plus proche de la zone de liaison 10 correspond au premier orifice 8A.

Ainsi, dans l'exemple illustré aux figures, la patte tibio-médiale 8 comporte trois orifices 8A, 8C, 8D, savoir un orifice proximal 8D, un orifice intermédiaire 8C et un orifice distal qui correspond au premier orifice 8A.

De préférence, afin de faciliter le positionnement de la tige 12B du premier organe d'ancrage 12 formant organe de fixation trans-articulaire, le premier orifice 8A est positionné sur la patte tibio-médiale 8 de façon à se trouver sensiblement au voisinage de la naissance de la protubérance osseuse formée par le pilon 1C. La demanderesse a en effet établi que cette position était particulièrement propice pour obtenir aisément une fixation stable et robuste par transpercement, à l'aide d'une seule et même tige 12B, du tibia 1 et du talus 2.

Afin de faciliter la pose du premier organe d'ancrage trans-articulaire 12, la première portée 8B du premier orifice 8A s'étend avantageusement selon un plan moyen P disposé en oblique relativement à la direction longitudinale principale d'extension Y-Y' de la patte tibio-médiale 8. En d'autres termes et comme illustré aux figures, le premier orifice 8A s'étend à travers l'épaisseur de la patte tibio-médiale 8 entre la face interne 13 de la patte 8 destinée à se trouver en contact avec l'os et la face externe 14 opposée, la première portée 8B étant disposée en oblique relativement à la face interne 13 et/ou à la face externe 14.

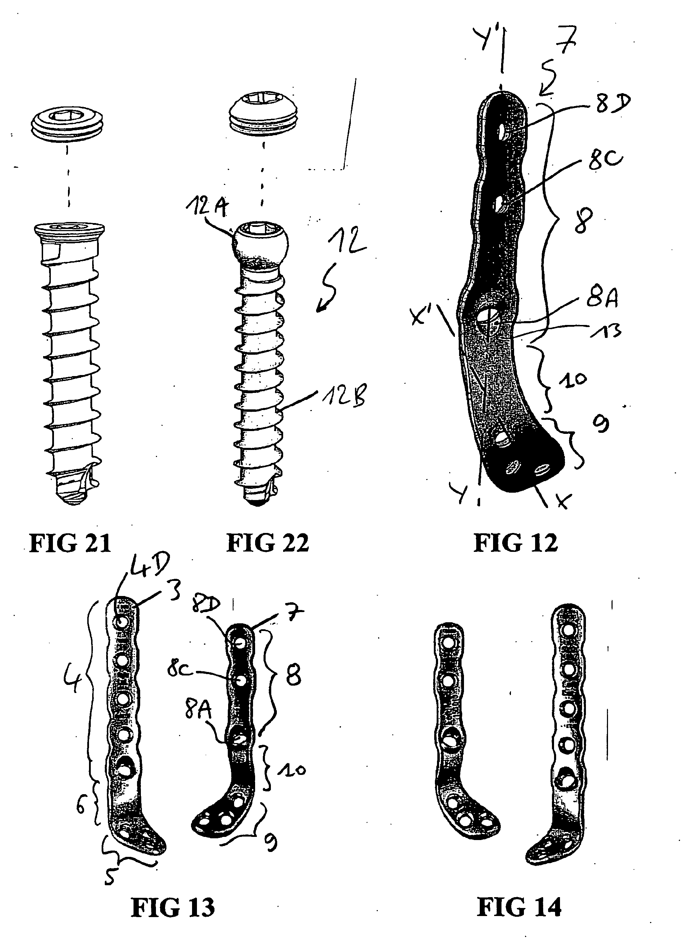

De préférence, la première portée 8B du premier orifice 8A présente une forme sensiblement arrondie, de préférence en forme de portion de sphère, pour permettre un réglage de l'orientation du premier organe d'ancrage 12 relativement à la patte tibio-médiale 8. A cette fin, la tête 12A du premier organe d'ancrage 12 présente également, au moins en ce qui concerne la partie de la tête 12A destinée à venir en contact avec la première portée 8B, une forme sphérique complémentaire (cf. figure 22).

Le chirurgien peut ainsi régler finement l'orientation du premier organe d'ancrage 12 relativement à la patte tibio-médiale 8 pour s'assurer, par exemple sous contrôle radiographique, que la tige 12B s'étend bien à travers à la fois le tibia 1 et le talus 2.

De préférence, afin de favoriser une bonne fixation osseuse, la tige 12B du premier organe d'ancrage 12 présente un diamètre (par exemple 3,5 mm) sensiblement supérieur au diamètre (par exemple 3 mm) des tiges des autres organes d'ancrage utilisés en coopération avec les autres orifices 8C, 8D ménagés à travers la patte tibio-médiale 6.

Avantageusement, la languette talo-médiale 9 est également pourvue d'orifices, de préférence au nombre de trois et disposés en triangle, la languette talo-médiale présentant avantageusement une forme élargie par rapport à la patte tibio-médiale 8 et à la zone de liaison 10. De préférence, ladite zone de liaison 8 est en revanche dépourvue d'orifice, ce qui facilite d'éventuelles opérations de pliage de la plaque que le chirurgien souhaiterait réaliser en cours d'intervention, pour s'adapter à des conditions anatomiques spécifiques.

La plaque antéro-médiale 7 comporte ainsi de préférence trois orifices de fixation alignés ménagés dans sa patte tibio-médiale 8 et trois orifices de fixation non alignés ménagés dans sa languette talo-médiale 9.

Comme on l'a vu dans ce qui précède, le premier orifice 8A présente avantageusement une portée sphérique 8B permettant de régler l'angulation de la vis avec laquelle il est destiné à coopérer, ladite portée sphérique 8B étant en outre orientée spécifiquement pour diriger la vis vers à la fois le tibia 1 et le talus 2, la vis en question (qui correspond au premier organe d'ancrage 12) formant ainsi une vis trans-articulaire.

De préférence, les autres orifices sont pourvus de portées tronconiques surmontées d'un filetage intérieur destiné à accueillir une contrevis permettant de bloquer la tête de vis (système de type Surfix® décrit en particulier dans la demande de brevet FR-07 04449 déposée le 21 juin 2007 et dans la demande de brevet européen EP-0 345 133 déposée le 26 mai 1989).

Avantageusement, comme décrit dans la demande de brevet FR-07 04449 précitée, chaque orifice ménagé dans la plaque antéro-médiale 7, à l'exception de préférence du premier orifice 8A, peut être utilisé indifféremment soit avec une vis à tête tronconique comme illustrée à la figure 21 (pour une fixation particulièrement ferme et stable), soit avec une vis à tête sphérique comme illustrée à la figure 22 (pour une fixation permettant un réglage de l'angulation de la vis par rapport à la plaque), l'usage dans tous les cas d'une contrevis permettant de solidariser efficacement les vis à la plaque.

De préférence, la plaque antéro-latérale 3 présente une conception sensiblement similaire à celle de la plaque antéro-médiale 5, à la différence près que la patte tibio-latérale 4 est de préférence plus longue que la patte tibio-médiale 6, et comporte plus d'orifices (cinq orifices destinés chacun à accueillir un organe de fixation, du genre vis, l'orifice distal, situé au plus près de la zone de liaison 6, présentant une portée disposée en oblique pour permettre à une vis 15 plus longue et plus grosse que les autres de traverser à la fois le tibia 1 et la talus 2.

Avantageusement, le système conforme à l'invention comprend en outre un moyen de compression 16 de l'articulation entre le tibia 1 et le talus 2, ledit moyen de compression 16 comportant :
- un premier mors 16A destiné à être fixé provisoirement au tibia 1,
- un deuxième mors 16B destiné à être fixé provisoirement à l'une ou l'autre des plaques antéro-latérale 3 et antéro-médiale 7
- et un moyen de rapprochement, de préférence manuel, des premier et second mors 16A, 16B l'un par rapport à l'autre.

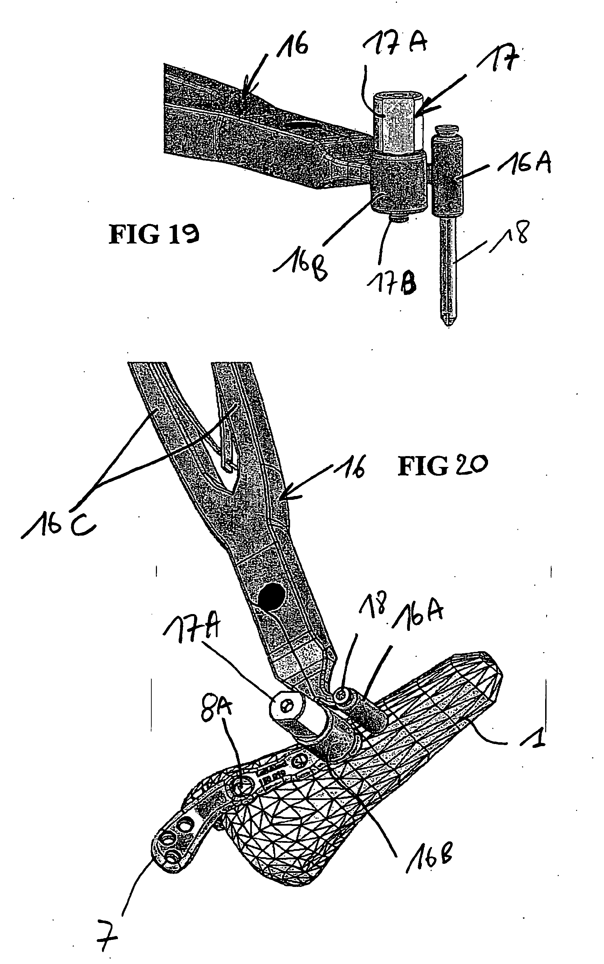

De préférence, la plaque antéro-latérale 3 et/ou la plaque antéro-médiale 7 comporte(nt) un trou fileté dans lequel est destiné à être vissé soit directement le deuxième mors 16B lui-même, soit une pièce indépendante 17 montée sur ledit deuxième mors 16B. Avantageusement, ledit trou fileté est ménagé dans la patte tibio-latérale 4 ou dans la patte tibio-médiale 8 (de préférence dans la patte tibio-latérale 4) et de façon encore plus préférentielle vers l'extrémité libre de la patte en question. De préférence et comme illustré aux figures, le moyen de compression 16 se présente sous la forme d'une pince, le deuxième mors 16B étant pourvu d'un manchon au sein duquel est enfilé un canon 17A pourvu d'un filetage extérieur 17B, ledit filetage extérieur 17B étant de préférence conçu pour être vissé soit dans le filetage intérieur de l'orifice proximal 4D ménagé dans la patte tibio-latérale 4, au plus près de l'extrémité libre de cette dernière, soit dans le filetage intérieur de l'orifice proximal 8D ménagé dans la patte tibio-médiale 8, au plus près de l'extrémité libre de cette dernière (cas illustré à la figure 20). Le deuxième mors est ainsi fixé à la patte tibio-latérale 4 ou à la patte tibio-médiale 8 par vissage dans l'orifice 4D, 8D concerné. Le filetage de l'orifice en question assume ainsi une double fonction puisqu'il permet d'une part d'assujettir le deuxième mors 16B du moyen de compression 16 à la plaque concerné, et qu'il permet d'autre part, une fois la compression réalisée, de coopérer avec une contrevis pour bloquer la tête de la vis de fixation insérée dans l'orifice en question (système Surfix® décrit dans les demandes FR-07 04449 et EP-0 345 133 précitées).

Bien entendu, l'invention concerne également en tant que telle et de manière indépendante le moyen de compression en question, de même qu'elle concerne de manière indépendante la plaque antéro-latérale 3 seule ainsi que la plaque antéro-médiale 5 seule.

L'utilisation du système conforme à l'invention va maintenant être décrit plus en détails.

En premier lieu, le patient est installé en position de supination sur une table d'opération radiologique. Une incision antérieure longitudinale de 10 à 12 cm est pratiquée latéralement directement sur le tendon antérieur du tibia. Le chirurgien pratique ensuite un nettoyage chirurgical afin d'exposer à la fois la face antérieure du tibia 1 et celle du talus 2. En particulier, le chirurgien enlève le cartilage du dôme du talus et du plafond tibial. La cas échéant, des greffons osseux peuvent être introduits dans l'articulation talo-tibiale.

Le chirurgien pose ensuite la plaque antéro-latérale 4 de préférence en premier. Pour cela, le chirurgien fixe tout d'abord la languette talo-latérale 5 sur le col 2A du talus 2, à l'aide de trois ensemble vis/contre-vis de type Surfix® tels qu'illustrés aux figures 21 et 22. La languette talo-latérale 5 est positionnée sur le côté latéral du col du talus 2, et est fixé à l'aide de trois vis qui sont introduites respectivement dans les trois orifices de fixation ménagés dans la languette talo-latérale 5.

Le chirurgien réalise ensuite la compression de l'articulation entre le tibia 1 et le talus 2.

A cette fin, le premier mors 16A de la pince constituant le moyen de compression 16 est attaché au tibia 1, à l'aide d'une vis provisoire 18, tandis que le deuxième mors 16B est vissé, par l'intermédiaire du canon 17A, dans le tibia 1, en alignement avec les orifices de fixation ménagés dans la patte tibio-latérale 4.

La pince de compression est ainsi fixée, en position ouverte, à la fois à la patte tibio-latérale 4 et au talon 2. Le chirurgien exerce alors un effort sur les bras 16C de la pince, ce qui conduit à un rapprochement des premier et second mors 16A, 16B l'un vers l'autre. Plus précisément, le premier mors 16A reste fixe tandis que le second mors 16B se rapproche du premier talus 2 auquel ladite plaque 4 est fixée. Le talus 2 est ainsi comprimé contre la base distale du tibia 1. L'articulation étant ainsi maintenue en compression, la patte tibio-latérale 4 est fixée définitivement au tibia 1 à l'aide de vis introduites dans les orifices ménagés dans la patte tibio-latérale 4.

Une fois la plaque antéro-latérale 3 ainsi fixée, le chirurgien introduit et fixe la plaque antéro-médiale 5 de manière similaire.

### POSSIBILITE D'APPLICATION INDUSTRIELLE

L'invention trouve son application industrielle dans l'élaboration, la fabrication et l'utilisation de systèmes pour immobiliser l'articulation talo-tibiale.

## Revendications

1. Système pour immobiliser l'articulation entre le tibia (1) et le talus (2) d'un patient humain, ledit système étant **caractérisé en ce qu'**il comprend au moins :
- une plaque antéro-latérale (3) comprenant elle-même une patte tiblo-latérale (4) et une languette talo-latérale (5) reliées par une zone de liaison (6), lesdites patte tibio-latérale (4), languette talo-latérale (5) et zone de liaison (6) étant conformées pour à la fois qu'au moins une portion de la patte tibio-latérale (4) repose sensiblement contre la face latérale antérieure (1A) du tibia (1) située vers l'extérieur du corps du patient par rapport à la crête tibiale antérieure (1 B) et que la languette talo-latérale (5) repose sensiblement contre le talus (2),
- une plaque antéro-médiale (7) comprenant elle-même une patte tibio-médiale (8) et une languette talo-médiale (9) reliées par une zone de liaison (10), lesdites patte tibio-médiale (8), languette talo-médiale (9) et zone de liaison (10) étant conformées pour à la fois qu'au moins une portion de la patte tibio-médiale (8) repose sensiblement contre la face médiale antérieure (1D) du tibia (1) située vers l'intérieur du corps du patient par rapport à la crête tibiale antérieure (1 B) et que la languette talo-latérale repose sensiblement contre le talus, ladite zone de liaison (10) de la plaque antéro-médiale (7) étant destinée à venir en regard d'une portion au moins du pilon tibial (1C), ladite zone de liaison (10) présentant une forme sensiblement complémentaire de celle de ladite portion du pilon tibial (1 C).

2. Système selon a revendication 1 **caractérisé en ce que** la plaque antéro-médiale (7) présente une forme sensiblement vrillée pour suivre le contour de ladite face médiale antérieure (1 D) du tibia (1) et du talus (2).

3. Système selon l'une des revendications 1 ou 2 **caractérisé en ce que** la patte tibio-médiale (8) présente une forme sensiblement arquée pour suivre le contour à la fois de ladite face médiale antérieure (1D) du tibia (1) et d'au moins une portion du pilon tibial (1C).

4. Système selon l'une des revendications 1 à 3 **caractérisé en ce que** la plaque antéro-médiale (7) présente, vue de profil, sensiblement une forme de cuiller.

5. Système selon l'une des revendications 1 à 4 **caractérisé en ce que** la languette talo-médiale (9) s'étend selon une direction longitudinale principale (X-X') qui est inclinée vers l'extérieur du corps du patient par rapport à la direction longitudinale principale d'extension (Y-Y') de la patte tibio-médiale (8).

6. Système selon l'une des revendications 1 à 5 **caractérisé en ce qu'**il comprend en outre au moins un premier organe d'ancrage (12) pourvu d'une tête (12A) et d'une tige d'ancrage osseux (12B) s'étendant à partir de ladite tête (12A), la patte tibio-médiale (8) comprenant au moins un premier orifice (8A) pourvue d'une première portée (8B) contre laquelle ladite tête (12A) est destinée à venir en butée, la portée (8B) étant conformée pour que la tige d'ancrage (12B) puisse s'étendre selon une direction lui permettant de traverser à la fois le tibia (1) et le talus (2).

7. Système selon la revendication 6 **caractérisé en ce que** ladite première portée (8B) s'étend selon un plan moyen (P) disposé en oblique relativement à la direction longitudinale principale d'extension (Y-Y') de la patte tibio-médiale (8).

8. Système selon la revendication 6 ou 7 **caractérisé en ce que** la première portée (8B) présente une forme sensiblement arrondie pour permettre un réglage de l'orientation du premier organe d'ancrage (12) relativement à la patte tibio-médiale (8).

9. Système selon l'une des revendications 6 à 8 **caractérisé en ce que** la patte tibio-médiale (8) comporte une pluralité d'orifices (8A, 8C, 8D) chacun destiné à être associé à un organe d'ancrage correspondant, l'orifice le plus proche de la zone de liaison (10) de la plaque antéro-médiale (7) correspondant audit premier orifice (8A).

10. Système selon la revendication 9 **caractérisé en ce que** la tige (12B) du premier organe d'ancrage (12) présente un diamètre sensiblement supérieur au diamètre des tiges des autres organes d'ancrage.

11. Système selon l'une des revendications 1 à 10 **caractérisé en ce qu'**il comprend un moyen de compression (16) de l'articulation entre le tibia (1) et le talus(2), ledit moyen de compression (16) comportant :
- un premier mors (16A) destiné à être fixé provisoirement au tibia (1),
- un deuxième mors (16B) destiné à être fixé provisoirement à l'une ou l'autre des plaques antéro-latérale (3) et antéro-médiale (7),
- et un moyen de rapprochement (16C), de préférence manuel, des premier et second mors (16A, 16B) l'un par rapport à l'autre.

12. Système selon la revendication 11 **caractérisé en ce que** la plaque antéro-latérale (3) et/ou la plaque antéro-médiale (7) comporte un trou fileté (4D, 8D) dans lequel est destiné à être vissé soit le deuxième mors (16B) lui-même, soit une pièce (17) montée sur le deuxième mors (16B).

13. Système selon la revendication 11 ou 12 **caractérisé en ce que** ledit trou fileté (4D, 8D) est ménagé dans la patte tibio-latérale (4) ou dans la patte tibio-médiale (8), de préférence vers l'extrémité libre de ladite patte.

## Patentansprüche

1. System zum Fixieren des Gelenks zwischen dem Schienbein (1) und dem Sprungbein (2) eines menschlichen Patienten, wobei das System **dadurch gekennzeichnet ist, dass** es mindestens Folgendes umfasst:
- eine vordere seitliche Platte (3), die ihrerseits eine bezüglich des Schienbeins seitliche Lasche (4) und eine bezüglich des Sprungbeins seitliche Zunge (5) umfasst, die durch einen Verbindungsbereich (6) verbunden sind, wobei die bezüglich des Schienbeins seitliche Lasche (4), die bezüglich des Sprungbeins seitliche Zunge (5) und der Verbindungsbereich (6) dafür ausgebildet sind, dass gleichzeitig wenigstens ein Abschnitt der bezüglich des Schienbeins seitlichen Lasche (4) im Wesentlichen an der seitlichen vorderen Seite (1A) des Schienbeins (1) anliegt, die sich in Bezug auf die vordere Schienbeinkante (1 B) an der Außenseite des Körpers des Patienten befindet, und die bezüglich des Sprungbeins seitliche Zunge (5) im Wesentlichen an dem Sprungbein (2) anliegt,
- eine vordere mediale Platte (7), die ihrerseits eine bezüglich des Schienbeins mediale Lasche (8) und eine bezüglich des Sprungbeins mediale Zunge (9) umfasst, die durch einen Verbindungsbereich (10) verbunden sind, wobei die bezüglich des Schienbeins mediale Lasche (8), die bezüglich des Sprungbeins mediale Zunge (9) und der Verbindungsbereich (10) dafür ausgebildet sind, dass gleichzeitig wenigstens ein Abschnitt der bezüglich des Schienbeins medialen Lasche (8) im Wesentlichen an der medialen vorderen Seite (1D) des Schienbeins (1) anliegt, die sich in Bezug auf die vordere Schienbeinkante (1 B) an der Innenseite des Körpers des Patienten befindet, und die bezüglich des Sprungbeins seitliche Zunge im Wesentlichen an dem Sprungbein anliegt, wobei der Verbindungsbereich (10) der vorderen medialen Platte (7) dazu bestimmt ist, in eine Position gegenüber einem Abschnitt mindestens des Pilon tibiale (1 C) zu gelangen, wobei der Verbindungsbereich (10) eine Form aufweist, die im Wesentlichen zu derjenigen dieses Abschnitts des Pilon tibiale (1 C) komplementär ist.

2. System nach Anspruch 1, **dadurch gekennzeichnet, dass** die vordere mediale Platte (7) eine Form aufweist, die im Wesentlichen so verwunden ist, dass sie der Kontur der medialen vorderen Seite (1D) des Schienbeins (1) und des Sprungbeins (2) folgt.

3. System nach einem der Ansprüche 1 oder 2, **dadurch gekennzeichnet, dass** die bezüglich des Schienbeins mediale Lasche (8) eine Form aufweist, die im Wesentlichen bogenförmig ist, so dass sie gleichzeitig der Kontur der medialen vorderen Seite (1 D) des Schienbeins (1) und wenigstens eines Abschnitts des Pilon tibiale (1 C) folgt.

4. System nach einem der Ansprüche 1 bis 3, **dadurch gekennzeichnet, dass** die vordere mediale Platte (7), von der Seite gesehen, im Wesentlichen die Form eines Löffels aufweist.

5. System nach einem der Ansprüche 1 bis 4, **dadurch gekennzeichnet, dass** sich die bezüglich des Sprungbeins mediale Zunge (9) in einer Hauptlängsrichtung (X-X') erstreckt, welche in Bezug auf die Hauptlängsrichtung (Y-Y') der Erstreckung der bezüglich des Schienbeins medialen Lasche (8) zur Außenseite des Körpers des Patienten hin geneigt ist.

6. System nach einem der Ansprüche 1 bis 5, **dadurch gekennzeichnet, dass** es außerdem mindestens ein erstes Verankerungselement (12) umfasst, das mit einem Kopfstück (12A) und einer sich von dem Kopfstück (12A) aus erstreckenden Knochenverankerungsstange (12B) versehen ist, wobei die bezüglich des Schienbeins mediale Lasche (8) mindestens eine erste Öffnung (8A) umfasst, die mit einem ersten Sitz (8B) versehen ist, an welchem das Kopfstück (12A) zur Anlage kommen soll, wobei der Sitz (8B) derart ausgebildet ist, dass sich die Verankerungsstange (12B) entlang einer Richtung erstrecken kann, die es ihr ermöglicht, sowohl das Schienbein (1) als auch das Sprungbein (2) zu durchqueren.

7. System nach Anspruch 6, **dadurch gekennzeichnet, dass** sich der erste Sitz (8B) entlang einer Mittelebene (P) erstreckt, die in Bezug auf die Hauptlängsrichtung (Y-Y') der Erstreckung der bezüglich des Schienbeins medialen Lasche (8) schräg angeordnet ist.

8. System nach Anspruch 6 oder 7, **dadurch gekennzeichnet, dass** der erste Sitz (8B) eine im Wesentlichen abgerundete Form aufweist, um eine Regelung der Ausrichtung des ersten Verankerungselements (12) in Bezug auf die bezüglich des Schienbeins mediale Lasche (8) zu ermöglichen.

9. System nach einem der Ansprüche 6 bis 8, **dadurch gekennzeichnet, dass** die bezüglich des Schienbeins mediale Lasche (8) mehrere Öffnungen (8A, 8C, 8D) aufweist, die jeweils dazu bestimmt sind, mit einem entsprechenden Verankerungselement verbunden zu werden, wobei die Öffnung, die dem Verbindungsbereich (10) der vorderen medialen Platte (7) am nächsten ist, der ersten Öffnung (8A) entspricht.

10. System nach Anspruch 9, **dadurch gekennzeichnet, dass** die Stange (12B) des ersten Verankerungselements (12) einen Durchmesser aufweist, der wesentlich größer als der Durchmesser der Stangen der anderen Verankerungselemente ist.

11. System nach einem der Ansprüche 1 bis 10, **dadurch gekennzeichnet, dass** es ein Mittel zum Zusammenpressen (16) des Gelenks zwischen dem Schienbein (1) und dem Sprungbein (2) umfasst, wobei das Mittel zum Zusammenpressen (16) aufweist:
- eine erste Spannbacke (16A), die dazu bestimmt ist, provisorisch an dem Schienbein (1) befestigt zu werden,
- eine zweite Spannbacke (16B), die dazu bestimmt ist, provisorisch entweder an der vorderen seitlichen (3) oder an der vorderen medialen Platte (7) befestigt zu werden,
- und ein Mittel (16C) zur vorzugsweise manuellen Annäherung der ersten und der zweiten Spannbacke (16A, 16B) aneinander.

12. System nach Anspruch 11, **dadurch gekennzeichnet, dass** die vordere seitliche Platte (3) und/oder die vordere mediale Platte (7) eine Gewindebohrung (4D, 8D) aufweist, das dazu bestimmt ist, dass entweder die zweite Spannbacke (16B) selbst oder ein an der zweiten Spannbacke (16B) angebrachtes Teil (17) in sie eingeschraubt wird.

13. System nach Anspruch 11 oder 12, **dadurch gekennzeichnet, dass** die Gewindebohrung (4D, 8D) in der bezüglich des Schienbeins seitlichen Lasche (4) oder in der bezüglich des Schienbeins medialen Lasche (8) ausgebildet ist, vorzugsweise zu dem freien Ende dieser Lasche hin.

## Claims

1. A system for immobilizing the joint between the tibia (1) and the talus (2) of a human patient, said system being **characterized in that** it comprises at least:
an antero-lateral plate (3), itself comprising a tibio-lateral strip (4) and a talo-lateral tab (5) interconnected by a link zone (6), said tibio-lateral strip (4), said talo-lateral tab (5), and said link zone (6) being shaped both so that: at least one portion of the tibio-lateral strip (4) rests substantially against the anterior lateral face (1A) of the tibia (1) that is situated towards the outside of the body of the patient relative to the anterior tibial crest (1 B); and also so that the talo-lateral tab (5) rests substantially against the talus (2); and
an antero-medial plate (7), itself comprising a tibio-medial strip (8) and a talo-medial tab (9) interconnected by a link zone (10), said tibio-medial strip (8), said talo-medial tab (9), and said link zone (10) being shaped both so that: at least one portion of the tibio-medial strip (8) rests substantially against the anterior medial face (1D) of the tibia (1) that is situated towards the inside of the body of the patient relative to the anterior tibial crest (1 B); and also so that the talo-medial tab rests substantially against the talus said link zone (10) of the antero-medial plate (7) being designed to come into register with at least a portion of the tibial pilon (1 C), said link zone (10) having a shape that is substantially complementary to the shape of said portion of the tibial pilon (1C).

2. A system according to claim 1, **characterized in that** the antero-medial plate (7) has a shape that is significantly twisted so as to match the outline of said anterior medial face (1 D) of the tibia (1) and of the talus (2);

3. A system according to any one of claims 1 or 2, **characterized in that** the tibio-medial strip (8) has a shape that is substantially arcuate so as to match both the outline of said anterior medial face (1 D) of the tibia (1) and the outline of at least one portion of the tibial pilon (1C).

4. A system according to any one of claims 1 to 3, **characterized in that** the antero-medial plate (7) has, as seen in profile, substantially the shape of a spoon.

5. A system according to any one of claims 1 to 4, **characterized in that** the talo-medial tab (9) extends in a main longitudinal direction (X-X') that is inclined towards the outside of the body of the patient relative to the main longitudinal direction (Y-Y') in which the tibio-medial strip (8) extends.

6. A system according to any one of claims 1 to 5, **characterized in that** it further comprises at least a first anchor member (12) provided with a head (12A) and with a bone anchor shank (12B) that extends from said head (12A), the tibio-medial strip (8) being provided with at least a first orifice (8A) provided with a first bearing surface (8B) against which said head (12A) is designed to come into abutment, the bearing surface (8B) being shaped so that the anchor shank (12B) can extend in a direction enabling it to pass both through the tibia (1) and through the talus (2).

7. A system according to claim 6, **characterized in that** said first bearing surface (8B) extends in a midplane (P) disposed slantingly relative to the main longitudinal direction (Y-Y') in which the tibio-medial strip (8) extends.

8. A system according to claim 6 or claim 7, **characterized in that** the first bearing surface (8B) has a substantially rounded shape so as to make it possible to adjust the orientation of the first anchor member (12) relative to the tibio-medial strip (8).

9. A system according to any one of claims 6 to 8, **characterized in that** the tibio-medial strip (8) is provided with a plurality of orifices (8A, 8C, 8D), each of which is designed to be associated with a corresponding anchor member, the orifice that is closest to the link zone (10) of the antero-medial plate (7) corresponding to said first orifice (8A).

10. A system according to claim 9, **characterized in that** the shank (12B) of the first anchor member (12) has a diameter that is significantly greater than the diameter of the shanks of the other anchor members.

11. A system according to any one of claims 1 to 10, **characterized in that** it further comprises compression means (16) for compressing the joint between the tibia (1) and the talus (2), said compression means (16) comprising:
a first jaw (16A) designed to be fastened temporarily to the tibia (1);
a second jaw (16B) designed to be fastened temporarily to either one of the plates, namely to the antero-lateral plate (3) or to the antero-medial plate (7); and
closure means (16C), which are preferably manual means, for bringing the first and second jaws (16A, 16B) closer together.

12. A system according to claim 11, **characterized in that** one or both of the antero-lateral plate (3) and the antero-medial plate (7) is/are provided with a tapped hole (4D, 8D) into which either the second jaw (16B) itself or a piece (17) mounted on the second jaw (16B) is designed to be screwed.

13. A system according to claim 11 or claim 12, **characterized in that** said tapped hole (4D, 8D) is provided in the tibio-lateral strip (4) or in the tibio-medial strip (8), preferably towards the free end of said strip.
